# EUROPEAN PATENT APPLICATION

(11) **EP 4 767 965 A1**
(43) Date of publication of application: **01.07.2026**
(21) Application number: 25217534.4
(22) Date of filing: 21.11.2025
(51) Int. Cl.: A61B 18/14

(54) **ELECTRODE RETENTION FEATURES FOR CATHETER INSTRUMENT**

(30) Priority: 26.12.2024 US 202419001739
(71) Applicant: Biosense Webster (Israel) Ltd., 2066717 Yokneam (IL)
(72) Inventor: WILCZYNSKI, Maribeth, Irvine, 92618 (US); MOLINA-GONZALEZ, Edwin, Irvine, 92618 (US)
(74) Representative: Carpmaels & Ransford LLP

(57) **Abstract**

An apparatus includes an elongate flexible shaft and an end effector. The shaft has a proximal end and a distal end. The end effector is positioned at a distal end of the shaft. The end effector includes a body, an electrode, and a bonding material. The electrode defines one or more laterally oriented openings. The bonding material secures the electrode to the body. A portion of the bonding material is disposed in at least one of the one or more laterally oriented openings.

## Description

### BACKGROUND

Cardiac arrhythmias, such as atrial fibrillation, occur when regions of cardiac tissue abnormally conduct electric signals. Procedures for treating arrhythmia include surgically disrupting the conducting pathway for such signals. By selectively applying electrical energy to cardiac tissue, it may be possible to cease or modify the propagation of unwanted electrical signals from one portion of the heart to another. Some such ablation treatments may include radio frequency (RF) ablation with alternating current (AC) electrical energy; and/or irreversible electroporation (IRE) via pulsed field direct current (DC) electrical energy (e.g., pulsed field ablation (PFA)). The ablation process may provide a barrier to unwanted electrical pathways by creating electrically insulative lesions or scar tissue that effectively block communication of aberrant electrical signals across the ablated tissue.

In some procedures, a catheter with one or more electrodes may be used to provide ablation within a patient. The catheter may be inserted into a major vein or artery (e.g., the femoral artery) and then advanced to position the electrodes within the heart or in a structure adjacent to the heart (e.g., the pulmonary vein). The one or more electrodes may be placed in contact with cardiac tissue or other vascular tissue and then activated with electrical energy to thereby ablate the contacted tissue (e.g., via RF energy, IRE, etc.). In some cases, the electrodes may be bipolar. In some other cases, a monopolar electrode may be used in conjunction with a ground pad or other reference electrode that is in contact with the patient. Irrigation may be used to draw heat from components of an ablation catheter; and to prevent the formation of blood clots near the tissue treatment site.

Examples of ablation catheters are described in U.S. Pat. No. 8,747,351, entitled "Catheter with Multi-Functional Control Handle Having Linear Mechanism," issued June 10, 2014, the disclosure of which is incorporated by reference herein, in its entirety; U.S. Pat. No. 8,956,353, entitled "Electrode Irrigation Using Micro-Jets," issued February 17, 2015, the disclosure of which is incorporated by reference herein, in its entirety; U.S. Pat. No. 9,220,433, entitled "Catheter with Variable Arcuate Distal Section," issued December 29, 2015, the disclosure of which is incorporated by reference herein, in its entirety; U.S. Pat. No. 10,130,422, entitled "Catheter with Soft Distal Tip for Mapping and Ablating Tubular Region," issued November 20, 2018, the disclosure of which is incorporated by reference herein, in its entirety; U.S. Pat. No. 10,702,177, entitled "Catheter with Bipole Electrode Spacer and Related Methods," issued July 7, 2020, the disclosure of which is incorporated by reference herein, in its entirety; U.S. Pat. No. 10,743,932, entitled "Integrated Ablation System using Catheter with Multiple Irrigation Lumens," issued August 18, 2020, the disclosure of which is incorporated by reference herein, in its entirety; and U.S. Pat. No. 11,559,349, entitled "Ablation Catheter with a Flexible Printed Circuit Board," issued January 24, 2023, the disclosure of which is incorporated by reference herein, in its entirety.

Some catheter ablation procedures may be performed after using electrophysiology (EP) mapping to identify tissue regions that should be targeted for ablation. Such EP mapping may include the use of sensing electrodes on a catheter (e.g., the same catheter that is used to perform the ablation or a dedicated mapping catheter). Such sensing electrodes may monitor electrical signals emanating from conductive endocardial tissues to pinpoint the location of aberrant conductive tissue sites that are responsible for the arrhythmia. Examples of an EP mapping system are described in U.S. Pat. No. 5,738,096, entitled "Cardiac Electromechanics," issued April 14, 1998, the disclosure of which is incorporated by reference herein, in its entirety. Examples of EP mapping catheters are described in U.S. Pat. No. 9,907,480, entitled "Catheter Spine Assembly with Closely-Spaced Bipole Microelectrodes," issued March 6, 2018, the disclosure of which is incorporated by reference herein, in its entirety; U.S. Pat. No. 10,130,422, entitled "Catheter with Soft Distal Tip for Mapping and Ablating Tubular Region," issued November 20, 2018, the disclosure of which is incorporated by reference herein, in its entirety; and U.S. Pat. No. 10,702,177, entitled "Catheter with Bipole Electrode Spacer and Related Methods," issued July 7, 2020, the disclosure of which is incorporated by reference herein, in its entirety.

Some catheter ablation procedures may be performed using an image guided surgery (IGS) system. The IGS system may enable the physician to visually track the location of the catheter within the patient, in relation to images of anatomical structures within the patient, in real time. Some systems may provide a combination of EP mapping and IGS functionalities, including the CARTO 3^{®} system by Biosense Webster, Inc. of Irvine, California. Examples of catheters that are configured for use with an IGS system are disclosed in U.S. Pat. No. 9,480,416, entitled "Signal Transmission Using Catheter Braid Wires," issued November 1, 2016, the disclosure of which is incorporated by reference herein, in its entirety; and various other references that are cited herein.

While several catheter systems and methods have been made and used, it is believed that no one prior to the inventors has made or used the invention described, illustrated and claimed herein.

### BRIEF DESCRIPTION OF THE DRAWINGS

The drawings and detailed description that follow are intended to be merely illustrative and are not intended to limit the scope of the invention as contemplated by the inventors.
FIG. 1 depicts a schematic view of a medical procedure in which a catheter of a catheter assembly is inserted in a patient.
FIG. 2 depicts a top plan view of the catheter assembly of FIG. 1, with an intermediate section of the catheter in a straight configuration shown in solid lines, and with the intermediate section of the catheter in a deflected configuration shown in broken lines.
FIG. 3 depicts a perspective view of an end effector of the catheter assembly of FIG. 1 and a distal portion of the catheter of FIG. 1.
FIG. 4 depicts a top plan view of the end effector of FIG. 3, with a first helical configuration shown in solid lines and a second helical configuration shown in broken lines.
FIG. 5 depicts a perspective view of an electrode of the end effector of FIG. 3.
FIG. 6 depicts a cross-sectional side view of a portion of the end effector of FIG. 3, showing a securement between the electrode of FIG. 5 and a body of the end effector.
FIG. 7 depicts a perspective view of an example of an alternative electrode that may be incorporated into the end effector of FIG. 3.
FIG. 8 depicts a cross-sectional side view of a portion of variation of the end effector of FIG. 3, showing a securement between the electrode of FIG. 7 and the body of the end effector.

### DETAILED DESCRIPTION FOR MODES OF CARRYING OUT THE INVENTION

The following description of certain examples of the invention should not be used to limit the scope of the present invention. The drawings, which are not necessarily to scale, depict selected embodiments and are not intended to limit the scope of the invention. The detailed description illustrates by way of example, not by way of limitation, the principles of the invention. Other examples, features, aspects, embodiments, and advantages of the invention will become apparent to those skilled in the art from the following description, which is by way of illustration, one of the best modes contemplated for carrying out the invention. As will be realized, the invention is capable of other different or equivalent aspects, all without departing from the invention. Accordingly, the drawings and descriptions should be regarded as illustrative in nature and not restrictive.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%. In addition, as used herein, the terms "patient," "host," "user," and "subject" refer to any human or animal subject and are not intended to limit the systems or methods to human use, although use of the subject invention in a human patient represents a preferred embodiment.

### I. Example of Catheter System

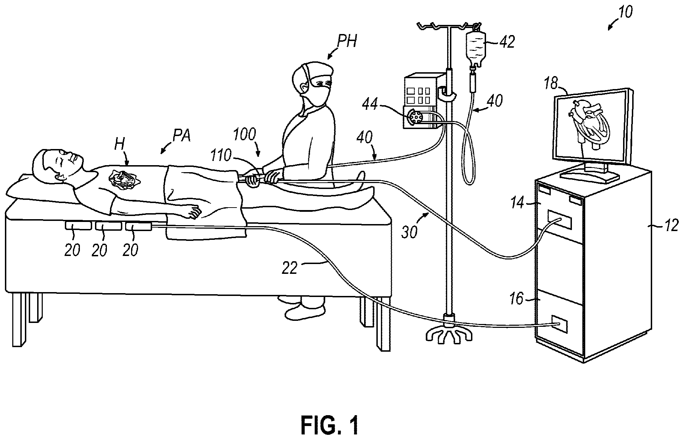

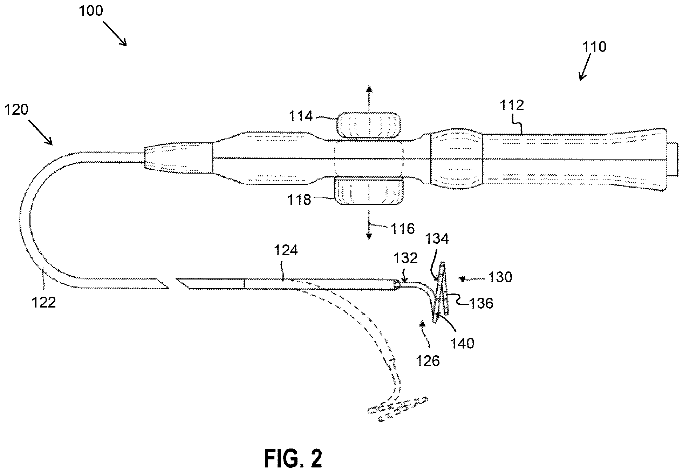

FIG. 1 shows an example of a medical procedure and associated components of a cardiac catheter system that may be used to provide EP mapping and/or cardiac ablation as referred to above. In particular, FIG. 1 shows a physician (PH) grasping a handle assembly (110) of a catheter assembly (100), with an end effector (130) (FIGS. 2-4) of a catheter (120) (FIG. 2) of catheter assembly (100) disposed in a patient (PA) to map potentials in tissue and/or ablate tissue in or near the heart (H) of the patient (PA). Catheter assembly (100) is coupled with a guidance and drive system (10) via a cable (30). Catheter assembly (100) is also coupled with a fluid source (42) via a fluid conduit (40). A set of field generators (20) are positioned underneath the patient (PA) and are coupled with guidance and drive system (10) via another cable (22).

Guidance and drive system (10) of the present example include a console (12) and a display (18). Console (12) includes a first driver module (14) and a second driver module (16). First driver module (14) is coupled with catheter assembly (100) via cable (30). In some variations, first driver module (14) is operable to receive EP mapping signals obtained via electrodes of end effector (130). Console (12) includes a processor (not shown) that processes such EP mapping signals and thereby provides EP mapping as is known in the art. In some other versions, end effector (130) does not provide EP mapping.

First driver module (14) of the present example is further operable to provide electrical power to electrodes (140) of end effector (130), as will be described in greater detail below, to thereby ablate tissue. In still other versions, end effector (130) does not provide ablation.

Second driver module (16) is coupled with field generators (20) via cable (22). Second driver module (16) is operable to activate field generators (20) to generate an alternating magnetic field around the heart (H) of the patient (PA). For instance, field generators (20) may include coils that generate alternating magnetic fields in a predetermined working volume that contains the heart (H).

First driver module (14) is also operable to receive position indicative signals from one or more navigation sensors (not shown) in end effector (130) and/or in catheter (120). In such versions, the processor of console (12) is also operable to process the position indicative signals from the one or more navigation sensors to thereby determine the position of end effector (130) within the patient (PA). In some versions, each navigation sensor includes one or more coils that are operable to generate signals that are indicative of the position and orientation of end effector (130) within the patient (PA). The coils are configured to generate electrical signals in response to the presence of an alternating electromagnetic field generated by field generators (20). Other components and techniques that may be used to generate real-time position data associated with end effector (130) may include wireless triangulation, acoustic tracking, optical tracking, inertial tracking, and the like. In some other variations, end effector (130) and/or catheter (120) lack a navigation sensor.

Display (18) is coupled with the processor of console (12) and is operable to render images of patient anatomy. Such images may be based on a set of preoperatively or intraoperatively obtained images (e.g., a CT or MRI scan, 3-D map, etc.). The views of patient anatomy provided through display (18) may also change dynamically based on signals from the navigation sensor assembly of end effector (130). For instance, as end effector (130) of catheter (120) moves within the patient (PA), the corresponding position data from the one or more navigation sensors may cause the processor of console (12) to update the patient anatomy views in display (18) in real time to depict the regions of patient anatomy around end effector (130) as end effector (130) moves within the patient (PA). Moreover, the processor of console (12) may drive display (18) to show locations of aberrant conductive tissue sites, as detected via electrophysiological (EP) mapping with end effector (130) or as otherwise detected (e.g., using a dedicated EP mapping catheter, etc.). By way of example only, the processor of console (12) may drive display (18) to superimpose the locations of aberrant conductive tissue sites on the images of the patient's anatomy, such as by superimposing an illuminated dot, a crosshair, or some other form of visual indication of aberrant conductive tissue sites.

The processor of console (12) may also drive display (18) to superimpose the current location of end effector (130) on the images of the patient's anatomy, such as by superimposing an illuminated dot, a crosshair, a graphical representation of end effector (130), or some other form of visual indication. Such a superimposed visual indication may also move within the images of the patient anatomy on display (18) in real time as the physician moves end effector (130) within the patient (PA), thereby providing real-time visual feedback to the operator about the position of end effector (130) within the patient (PA) as end effector (130) moves within the patient (PA). The images provided through display (18) may thus effectively provide a video tracking the position of end effector (130) within a patient (PA), without necessarily having any optical instrumentation (i.e., cameras) viewing end effector (130). In the same view, display (18) may simultaneously visually indicate the locations of aberrant conductive tissue sites detected through EP mapping. The physician (PH) may thus view display (18) to observe the real time positioning of end effector (130) in relation to the mapped aberrant conductive tissue sites and in relation to images of the adjacent anatomical structures in the patient (PA).

Fluid source (42) of the present example includes a bag containing saline or some other suitable irrigation fluid. Conduit (40) includes a flexible tube that is further coupled with a pump (44), which is operable to selectively drive fluid from fluid source (42) to catheter assembly (100). Such irrigation fluid may be expelled through openings (not shown) of end effector (130). Such irrigation may be provided in any suitable fashion as will be apparent to those skilled in the art in view of the teachings herein.

### II. Example of Catheter Assembly

FIGS. 2-4 show catheter assembly (100) in greater detail. In the example shown, and as noted above, catheter assembly (100) includes a handle assembly (110), a catheter (120) extending distally from handle assembly (110), and an end effector (130) at the distal end of catheter (120).

Handle assembly (110) of the present example includes a handle housing (112) that houses components of a deflection control assembly, the operation of which will be described in greater detail below. This deflection control assembly includes a rotary actuator (114) that can be directly manipulated by an operator by rotating actuator (114) about an axis (116). Axis (116) is generally transverse or perpendicular to the longitudinal axis of handle housing (112). A deflection tension adjustment dial (118) opposes actuator (114) along axis (115). Deflection tension adjustment dial (118) is coupled to, and indirectly engaged with, actuator (114) by various mechanisms and parts and thereby allows an operator to adjust the ease with which actuator (114) may be rotated. While actuator (114) is operated via rotation, the resistance to which is adjustable via adjustment dial (118), other suitable features may be used to provide control of the deflection control assembly of handle assembly (110) as will be apparent to those skilled in the art in view of the teachings herein.

Catheter (120) of the present example includes an elongate catheter body (122), a deflectable intermediate section (124) at a distal end of catheter body (122), and a tip section (126) at a distal end of intermediate section (124). End effector (130) is provided at tip section (126). As noted above, handle assembly (110) includes components of a deflection control assembly. This deflection control assembly is further coupled with intermediate section (124) of catheter (120) via one or more pull-wires, cables, bands, and/or other components. The deflection control assembly is operable to laterally deflect catheter (120) at intermediate section (124) in response to rotation of actuator (114). An example of such deflection is shown in FIG. 2, where intermediate section (124) is shown in a straight configuration in solid lines; and in an example of a deflected configuration in broken lines. Such controllable deflection at intermediate section (124) may facilitate positioning of end effector (130) at a targeted tissue region.

By way of example only, controlled deflection of catheter (120) at intermediate section (124) may be provided in accordance with at least some of the teachings of U.S. Pat. No. 8,747,351, entitled "Catheter with multi-functional control handle having linear mechanism," issued June 10, 2014, the disclosure of which is incorporated by reference herein. In some other versions, catheter assembly (100) lacks a feature providing controlled deflection of catheter (120) at intermediate section (124).

End effector (130) of the present example includes a generally straight proximal region (132) and a generally circular main region (134). Generally circular main region (134) may form a flat circle or may be at least slightly helical as described in greater detail below. End effector (130) includes a body (136) in the form of electrically insulative tubing, which may have a round cross-sectional shape or any other suitable cross-sectional shape. By way of example only, body (136) may comprise a poly(etherurethane) material (e.g., PELLETHANE thermoplastic polyurethane by the Lubrizol Corporation of Wickliffle, Ohio) and/or any other suitable material(s). A series of electrodes (140) are mounted on body (136) along generally circular main region (134), in a longitudinally spaced-apart arrangement. In the present example, generally circular main region (134) defines, when unconstrained, a generally helical form. In some versions or scenarios, this helical form is centered along the longitudinal axis (125) of intermediate section (124). In some other versions or scenarios, this helical form is oriented obliquely relative to the longitudinal axis (125) of intermediate section (124).

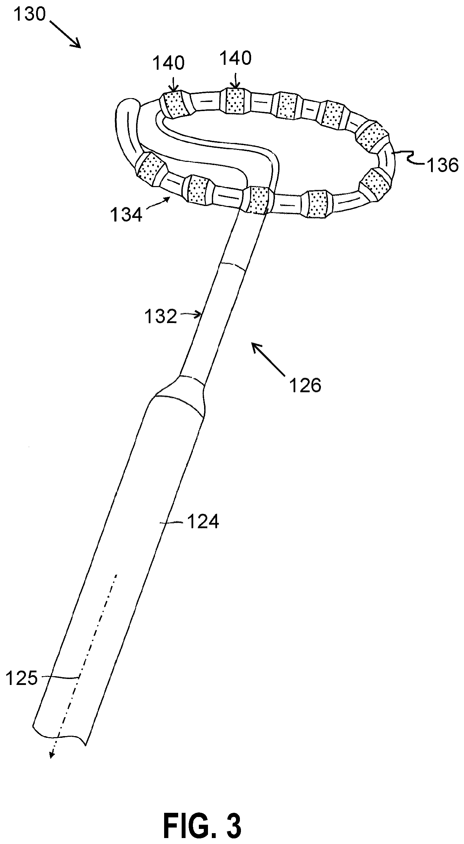

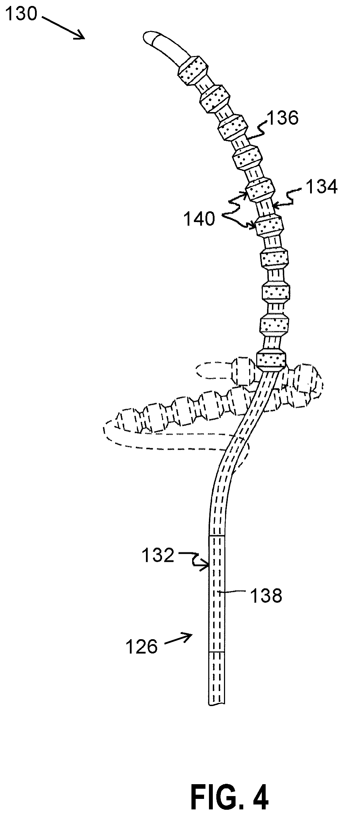

In the present example, catheter assembly (100) is operable to provide controlled contraction of the helical form defined by circular main region (134), thereby providing controlled variation of the radius and/or pitch of the helical form. An example of controlled variation of the helical form of circular main region (134) is shown in FIG. 3. Specifically, FIG. 4 shows circular main region (134) having a coarser pitch in solid lines; and a finer pitch in broken lines. In some versions, this control is provided via an actuator (not shown) of handle assembly (110) that is operable to drive longitudinal movement of a mandrel (138) (FIG. 4), which is slidably disposed within body (136) of end effector (130). By way of further example only, the control over the helical form defined by circular main region (134) may be provided via one or more pull wires and/or via any other suitable components.

In some variations, controlled contraction of the helical form defined by circular main region (134) may be provided in accordance with at least some of the teachings of U.S. Pat. No. 9,220,433, entitled "Catheter with Variable Arcuate Distal Section," issued December 29, 2015, the disclosure of which is incorporated by reference herein, in its entirety. In some other versions, catheter assembly (100) lacks a feature providing controlled contraction of the helical form defined by circular main region (134).

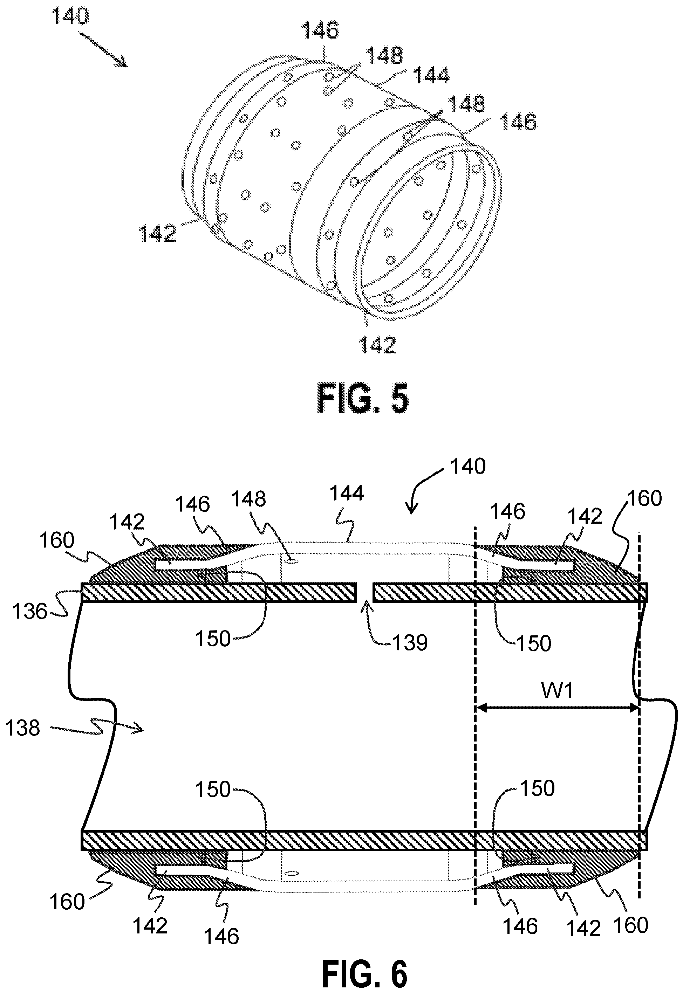

FIG. 5 shows electrode (140) in further detail. Electrode (140) of this example is generally ring-shaped or barrel-shaped. Electrode (140) comprises an electrically conductive material such that electrode (140) is operable to apply electrical energy to tissue as described above and as described in various references cited herein. By way of example only, electrode (140) may comprise a noble metal, such as gold, platinum, ruthenium, rhodium, palladium, silver, osmium, iridium, etc. Alternatively, any other suitable electrically conductive material(s) may be used. In some versions, electrode (140) is also operable to pick up electrical potentials in tissue (e.g., as part of an EP mapping process), as also described above and as described in various references cited herein. Electrode (140) of the present example includes annular end portions (142), a central portion (144), and transition portions (146). Each end portion (142) has a first inner diameter and first outer diameter. Central portion (144) has a second inner diameter and a second outer diameter. The second inner diameter is larger than the first inner diameter. Similarly, the second outer diameter is larger than the second outer diameter. Transition portions (146) provide substantially smooth transitions from these different diameters of end portions (142) and central portion (144). In some versions, each transition portion provides a straight, angled/tapered surface transition between central portion (144) and the respective end portion (142). In some other versions, each transition portion (146) provides a curved or contoured surface transition between central portion (144) and the respective end portion (142).

Electrode (140) of the present example further includes a plurality of irrigation openings (148) formed through central portion (148). Irrigation openings (148) are oriented laterally in the present example. In some versions, transition portions (146) also define irrigation openings (148). Irrigation openings (148) may be used to communicate irrigation fluid outwardly from electrodes (140), as described above and as described in various references cited herein. Such irrigation fluid may prevent overheating of electrodes (140), may prevent buildup of coagulum on electrodes (140), may promote electrical conductivity of electrodes (140), and/or may provide other effects.

As shown in FIG. 6, the irrigation fluid may be communicated to electrode (140) via a lumen (138) defined in body (136) of end effector (130). Lumen (138) is in fluid communication with the interior of electrode (140) via one or more lateral ports (139), such that irrigation fluid may flow through lumen (138) and into the interior of electrode (140) via lateral port(s) (139); then flow out of electrode (140) via irrigation openings (148). While lumen (138) is shown in FIG. 6 as spanning along an entire inner diameter of body (136), this is merely a schematic representation. Lumen (138) may in fact be substantially smaller than what is shown in FIG. 6, and various other components or structures may be positioned in body (136). Moreover, some variations of electrode (140) may lack irrigation openings (148). In some such variations, lumen (138) is omitted.

As also shown in FIG. 6, electrode (140) is secured to body (136) via a bonding material (160). By way of example only, bonding material (160) may comprise polyurethane and/or any other suitable material(s). Bonding material (160) is applied at each end portion (142) of electrode (140) and along the adjacent region of body (136). Bonding material (160) may be applied to electrode (140) and body (136) while bonding material (160) is in a flowable form (e.g., liquid, etc.), and then bonding material (160) may later cure to a solid form. As shown in FIG. 6, the inner diameter of each end portion (142) is larger than the outer diameter of body (136), such that bonding material (160) flows into a gap (150) defined between the inner diameter of each end portion (142) and the outer diameter of body (136).

Bonding material (160) also covers the entire inner and outer surfaces of each end portion (142). In addition, bonding material (160) covers at least a portion of each transition portion (146). In some versions, bonding material (160) covers more than 50% of the external surface area of each transition portion (146). Bonding material (160) nevertheless leaves central portion (144) exposed, such that bonding material (160) does not substantially interfere with the electrical communication and irrigation functionality of electrode (140).

In addition to providing structural securement of electrode (140) to body (136), bonding material (160) also provides a fluid-tight seal at end portions (142) of electrodes (140), thereby ensuring that irrigation fluid communicated to the interior of electrodes (140) only exits electrodes (140) via irrigation openings (148). In the present example, bonding material (160) spans longitudinally along a first width (W1) at each end of electrode (140), such that bonding material (160) does not extend along body (136) from end portion (142) of one electrode (140) to end portion (142) of another electrode (140). In some other versions, each region of bonding material (160) extends along body (136) from end portion (142) of one electrode (140) to end portion (142) of another electrode (140), while still leaving central portion (144) of each electrode (140) exposed.

### III. Example of Alternative Electrode and Securement

During operation of catheter assembly (100), end effector (130) may be bent numerous times in numerous different ways. For instance, end effector (130) may bend numerous times in numerous different ways as end effector (130) traverses tortuous passageways en route to the target site within the patient (PA). End effector (130) may also bend numerous times in numerous different ways as the physician (PH) drives end effector (130) to vary the radius and/or pitch of the helical form defined by circular main region (134) as described above with reference to FIG. 4. In some instances, bending of end effector (130) may create stress (e.g., tensile stress, compressive stress, torsional stress, etc.) at the margins between bonding material (160) and body (136) and/or at the margins between bonding material (160) and electrode (140). Such stress may eventually result in bonding material (160) delaminating from body (136) and/or electrode (140). Such delamination of bonding material may eventually result in blood ingress into the interior region of electrode (140), which may in turn eventually result in formation of coagulum within electrode (140), which may in turn adversely affect the electrical communication and/or irrigation capabilities of electrode (140).

To enhance the securement between electrode (140) and body (136) and/or otherwise reduce the risk of delamination of bonding material (160), there may be a tendency to simply use more bonding material (160) to secure electrode (140) to body (136). However, this solution may have several drawbacks. For instance, providing more bonding material (160) along body (136) may increase rigidity of end effector (130), which may make it more difficult to navigate end effector (130) along tortuous passageways and/or achieve desired helical forms in circular main region (134). Providing more bonding material (160) along electrode (140) may reduce the electrically active surface of electrode (140), which may in turn increase electrical current density of electrode (140), which may in turn increase the risk of arcing, charring, coagulum formation, and/or other undesirable results.

It may therefore be desirable to provide an alternative securement between electrode (140) and body (136) that reduces the risk of delamination while avoiding adverse effects such as increasing the rigidity of end effector (130) and/or adversely impacting the electrical performance of electrodes.

FIGS. 6-7 show an example of an alternative electrode (240) and alternative securement of electrode (240) to body (136) that may provide the advantages described above while avoiding the adverse effects described above. Electrode (240) of this example may be configured and operable like electrode (140) except for the differences noted below. Electrode (240) may thus be incorporated into end effector (130) as a substitute for elecrdoe (140).

Electrode (240) of the present example is generally ring-shaped or barrel-shaped, such that electrode (240) constitutes a generally cylindrical member extending from one annular end portion (242) to another annular end portion (242). Electrode (240) comprises an electrically conductive material such that electrode (240) is operable to apply electrical energy to tissue as described above and as described in various references cited herein. By way of example only, electrode (240) may comprise a noble metal, such as gold, platinum, ruthenium, rhodium, palladium, silver, osmium, iridium, etc. Alternatively, any other suitable electrically conductive material(s) may be used. In some versions, electrode (240) is also operable to pick up electrical potentials in tissue (e.g., as part of an EP mapping process), as also described above and as described in various references cited herein. Electrode (240) of the present example includes annular end portions (242), a central portion (244), and transition portions (246) arranged in a longitudinally extending set, with central portion (244) being longitudinally centered between end potions (242) and transition portions (246). Each end portion (242) has a first inner diameter, defined by an inner surface about a central longitudinal axis (LA); and first outer diameter. Central portion (244) has a second inner diameter, defined by an inner surface about the central longitudinal axis (LA); and a second outer diameter, defined by an outer surface about the central longitudinal axis (LA). The second inner diameter is larger than the first inner diameter. Similarly, the second outer diameter is larger than the second outer diameter.

By way of example only, the second inner diameter may range from approximately 1.50 mm to approximately 3.50 mm; from approximately 2.00 mm to approximately 3.00 mm; or more particularly may be approximately 2.62 mm. Alternatively, the second inner diameter may be any other suitable size. By way of further example only, electrode (240) may have a length (along the central longitudinal axis (LA)) ranging from approximately 1.00 to approximately 5.00 mm; ranging from approximately 2.00 mm to approximately 4.00 mm; or more particularly may be approximately 3.00 mm. Alternatively, electrode (240) may have any other suitable length.

Transition portions (246) provide substantially smooth transitions from these different diameters of end portions (242) and central portion (244). In some versions, each transition portion provides a straight, angled/tapered surface transition between central portion (244) and the respective end portion (242). In some other versions, each transition portion (246) provides a curved or contoured surface transition between central portion (244) and the respective end portion (242).

Electrode (240) of the present example further includes a plurality of irrigation openings (248) formed through central portion (248). Irrigation openings (248) are oriented laterally in the present example. In some versions, transition portions (246) also define irrigation openings (248). Irrigation openings (248) may be used to communicate irrigation fluid outwardly from electrodes (240), as described above and as described in various references cited herein. Such irrigation fluid may prevent overheating of electrodes (240), may prevent buildup of coagulum on electrodes (240), may promote electrical conductivity of electrodes (240), and/or may provide other effects.

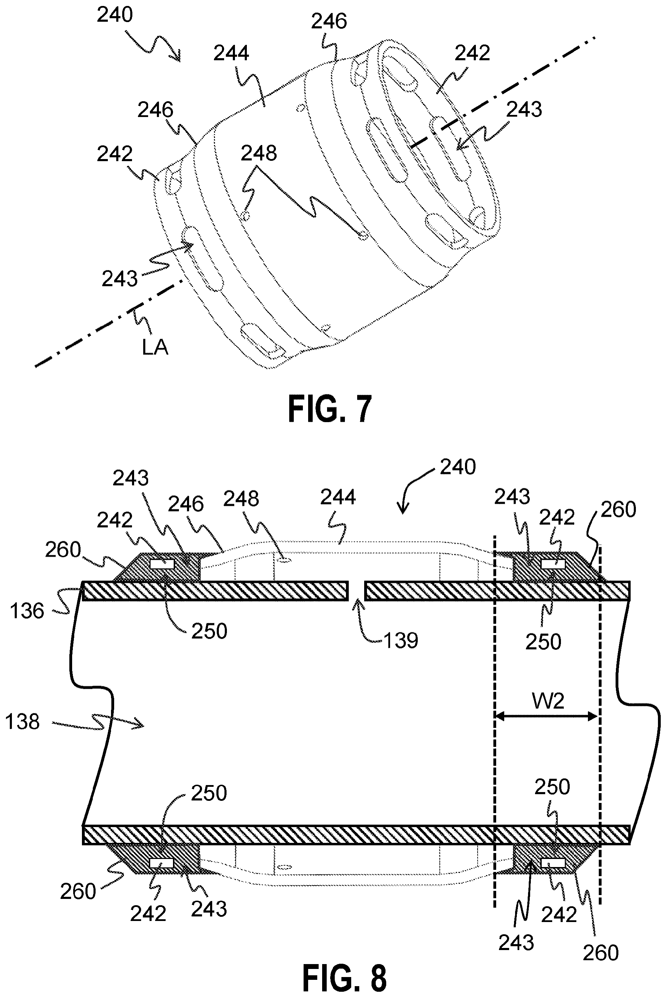

As shown in FIG. 8, the irrigation fluid may be communicated to electrode (240) via lumen (138) and lateral port(s) (139) of body (136) of end effector (130). As noted above with reference to FIG. 6, while lumen (138) is shown in FIG. 8 as spanning along an entire inner diameter of body (136), this is merely a schematic representation. Lumen (138) may in fact be substantially smaller than what is shown in FIG. 8, and various other components or structures may be positioned in body (136). Moreover, some variations of electrode (240) may lack irrigation openings (248). In some such variations, lumen (138) is omitted.

Unlike electrode (140), electrode (240) of the present example further includes a set of laterally oriented openings (243) formed along each end portion (242). Each opening (243) is elongate and extends circumferentially along a part of end portion (242), such that openings (243) constitute a form of circumferential cutouts. Openings (243) of each set are angularly spaced apart from each other equidistantly along end portion (242) about a central longitudinal axis (LA). In the present example, openings (243) are formed only in end portions (242), such that openings (243) do not extend into transition portions (246). In some other versions, at least a portion of one or more openings (243) extends into an adjacent transition portion (246).

By way of further example only, each opening (243) may have a width (measured along a dimension parallel with the central longitudinal axis (LA)) ranging from approximately 0.05 mm to approximately 0.35 mm; from approximately 0.10 mm to approximately 0.30 mm; or more particularly may be approximately 0.20 mm. Alternatively, each opening (243) may have any other suitable width. In addition, each opening (243) may have a length (measured along a circumferential dimension extending angularly about the central longitudinal axis (LA)) ranging from approximately 0.20 mm to approximately 0.60; or from approximately 0.30 mm to approximately 0.50 mm; or more particularly approximately 0.40 mm. Alternatively, each opening (243) may have any other suitable length.

FIG. 8 shows how openings (243) of electrode (240) facilitate securement of electrode (240) to body (136). Electrode (240) is secured to body (136) via a bonding material (260). By way of example only, bonding material (260) may comprise polyurethane and/or any other suitable material(s). Bonding material (260) is applied at each end portion (242) of electrode (240) and along the adjacent region of body (136). Bonding material (260) may be applied to electrode (240) and body (136) while bonding material (260) is in a flowable form (e.g., liquid, etc.), and then bonding material (260) may later cure to a solid form. As shown in FIG. 8, the inner diameter of each end portion (242) is larger than the outer diameter of body (136), such that bonding material (260) flows into a gap (250) defined between the inner diameter of each end portion (242) and the outer diameter of body (136).

Bonding material (260) also covers the entire inner and outer surfaces of each end portion (242). In addition, bonding material (260) flows through each opening (243), such that portions of bonding material (260) effectively wrap around the entire outer region of each end portion (242) adjacent to openings (243). In some cases, some bonding material (260) may also cover part of transition portion (246) adjacent to openings (243). In some other versions, bonding material (260) does not cover any part of transition portion (246) adjacent to openings (243). In either case, bonding material (260) may leave central portion (244) entirely exposed, such that bonding material (260) does not substantially interfere with the electrical communication and irrigation functionality of electrode (240).

In addition to providing structural securement of electrode (240) to body (136), bonding material (260) also provides a fluid-tight seal at end portions (242) of electrodes (240), thereby ensuring that irrigation fluid communicated to the interior of electrodes (240) only exits electrodes (240) via irrigation openings (248). In the present example, bonding material (260) spans longitudinally along a second width (W2) at each end of electrode (240), such that bonding material (260) does not extend along body (136) from end portion (242) of one electrode (240) to end portion (242) of another electrode (240). In some other versions, each region of bonding material (260) extends along body (136) from end portion (242) of one electrode (240) to end portion (242) of another electrode (240), while still leaving central portion (244) of each electrode (240) exposed.

In comparing the securement of electrode (240) to body (136) via bonding material (260) with the securement of electrode (140) to body (136) via bonding material (160), it should be noted that the presence of openings (243), and the flowing of bonding material (260) through openings (243), may provide a lower risk of delamination. This may be due in part to a tendency of bonding material (160, 260) to adhere better to the material of body (136) (e.g., a polymeric material) than to the material of electrode (140, 240) (e.g., a metallic material). In the context of electrode (140), the entire interface between electrode (140) and bonding material (160) may be vulnerable to shearing stresses, which may be regularly encountered during normal use of end effector (130). By contrast, in the context of electrode (240), the formation of a loops by bonding material (260) flowing through each opening (243) and adjacent regions of end portions (242) may substantially reduce if not eliminate the vulnerability to shearing stresses. In other words, bonding material (260) fully encapsulates the regions of end portions (242) between openings (243) and the adjacent outer edges of end portions (242). To analogize with a grasp of a hand on electrodes (140, 240), the securement between bonding material (260) and electrode (240) is akin to the fingers of the hand wrapping fully around a portion of electrode (240) to fully grasp electrode (240); while the securement between bonding material (160) and electrode (140) is akin to the fingers of the hand simply pinching a portion of electrode (140). The enhanced securement between bonding material (260) and electrode (240) allows bonding material (260) to bear more stress and redistribute more stress to body (136) than the stress that is otherwise applied at the interface between bonding material (160) and electrode (140).

By providing an enhanced securement due to the flow of bonding material (260) through openings (134), the arrangement shown in FIG. 8 also allows less bonding material (260) to be used than the amount of bonding material (160) used in the arrangement shown in FIG. 6. This is demonstrated by the second width (W2) being smaller than the first width (W1). With the second width (W2) being smaller, more surface area of electrode (240) may be left exposed by bonding material (260), as compared to the surface area of electrode (140) that is left exposed by bonding material (160). This may allow electrode (240) to provide better electrical communication with tissue than electrode (140). Thus, the arrangement shown in FIG. 8 may provide a combination of (i) a reduced risk of delamination of bonding material (260), and (ii) enhanced electrical communication between tissue and electrode (240), which are two results that might otherwise seem at odds with each other to those skilled in the art.

In summary, the invention described herein may provide one or more of the following advantages (among others): (i) providing a riveting effect that enhances attachment of electrodes (240) relative to body (136); (ii) partially redistributing the stress on bonding material (260) (e.g., polyurethane, etc.) towards the openings (243) and away from the margins and consequently reducing the stress on the bonding material (260) margins, which may in turn reduce the potential for delamination; and/or (iii) openings (243) may be used as a visual guide for improved consistency of bonding material (260) margins. In addition, if the use of openings (243) reduces the possibility of delamination, then the width of the bonding material (260) margins could be reduced, thus allowing for larger active electrode surface for each electrode (240). Consequently, this may reduce the applied current density and therefore the potential for arcing and creation of char on the ablated tissue.

### IV. Examples of Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

A ring electrode for pulsed field ablation comprising: (a) a generally cylindrical member extending from one annular end portion to another annular end portion with a central portion including a first transition portion disposed between the central portion and the one annular portion and a second transition portion disposed between the central portion and the another annular end portion, the central portion including an inner diameter defining an inner surface about the longitudinal axis and an outer diameter defining the outer surface about the longitudinal axis, each of the annular end portions including an inner diameter smaller than the inner diameter of the central portion; and (b) a plurality of circumferential cut outs disposed on one of the annular end portions or the transition portions.

### Example 2

The ring electrode of Example 1, in which the plurality of circumferential cut outs are disposed on the annular end portions.

### Example 3

The ring electrode of Example 1 in which the plurality of circumferential cut outs are disposed on a portion of the annular end portion and the transition portion.

### Example 4

The ring electrode of Example 1 in which each of the cut out defines a circumferential slot having a width of approximately 0.20 mm and a length of approximately 0.40 mm.

### Example 5

An apparatus, comprising: (a) an elongate flexible shaft having a proximal end and a distal end; and (b) an end effector positioned at a distal end of the shaft, the end effector including: (i) a body, (ii) an electrode, the electrode defining one or more laterally oriented openings, and (iii) a bonding material securing the electrode to the body, a portion of the bonding material being disposed in at least one of the one or more laterally oriented openings.

### Example 6

The apparatus of Example 5, the electrode including a pair of outer portions and a central portion longitudinally interposed between the outer portions.

### Example 7

The apparatus of Example 7, the bonding material being positioned over at least part of each outer portion of the pair of outer portions.

### Example 8

The apparatus of any of Examples 6 through 7, the at least one of the one or more laterally oriented openings in which the bonding material is disposed being positioned on an outer portion of the pair of outer portions.

### Example 9

The apparatus of Example 8, each outer portion of the pair of outer portions including a respective plurality of laterally oriented openings, the one or more laterally oriented openings of the electrode including the plurality of laterally oriented openings of the outer portions.

### Example 10

The apparatus of any of Examples 6 through 9, each outer portion of the pair of outer portions defining a gap with an outer surface of the body.

### Example 11

The apparatus of Example 10, a portion of the bonding material being disposed in the gap.

### Example 12

The apparatus of any of Examples 10 through 11, the gap being positioned adjacent to the at least one of the one or more laterally oriented openings in which the bonding material is disposed.

### Example 13

The apparatus of any of Examples 6 through 12, each outer portion of the pair of outer portions having a first outer diameter, the central portion having a second outer diameter.

### Example 14

The apparatus of Example 13, the second outer diameter being larger than the first outer diameter.

### Example 15

The apparatus of any of Examples 13 through 14, the electrode further including a pair of transition portions, each transition portion being longitudinally interposed between the central portion and a respective outer portion of the pair of outer portions.

### Example 16

The apparatus of Example 15, the transition portions providing a smooth transition from the first outer diameter to the second outer diameter.

### Example 17

The apparatus of any of Examples 13 through 16, the transition portions and the central portion being free of the bonding material.

### Example 18

The apparatus of any of Examples 6 through 16, the central portion being free of the bonding material.

### Example 19

The apparatus of any of Examples 5 through 18, the one or more laterally oriented openings further including at least one irrigation opening configured to expel irrigation fluid from the electrode.

### Example 20

The apparatus of Example 19, the body defining a lumen and a lateral port, the lumen and the lateral port being configured to communicate irrigation fluid to the electrode for expulsion via the at least one irrigation opening.

### Example 21

The apparatus of any of Examples 5 through 20, the end effector further comprising a plurality of additional electrodes, the additional electrodes being longitudinally spaced apart from each other along the body, each electrode of the plurality of additional electrodes respectively defining one or more laterally oriented openings, the bonding material of the end effector further securing the additional electrodes to the body, respective portions of the bonding material being disposed in at least one of the one or more laterally oriented openings of the respective additional electrodes.

### Example 22

The apparatus of Example 21, further comprising an actuator operable to drive the body between a first helical configuration and a second helical configuration.

### Example 23

The apparatus of any of Examples 5 through 22, further comprising a handle assembly, the shaft extending distally from the handle assembly.

### Example 24

The apparatus of any of Examples 5 through 23, the shaft including a steerable section, the apparatus further including an actuator operable to drive lateral deflection of the steerable section of the shaft.

### Example 25

The apparatus of any of Examples 5 through 24, the electrode being operable to apply electrical energy to tissue.

### Example 26

The apparatus of any of Examples 5 through 25, the bonding material comprising polyurethane.

### Example 27

The apparatus of any of Examples 5 through 26, the body comprising poly(etherurethane).

### Example 28

The apparatus of any of Examples 5 through 27, the electrode comprising a noble metal.

### Example 29

An apparatus, comprising: (a) an elongate flexible shaft having a proximal end and a distal end; and (b) an end effector position.ned at a distal end of the shaft, the end effector including: (i) a body, (ii) a plurality of electrodes longitudinally spaced apart from each other along the body, each electrode of the plurality of electrodes including: (A) a first outer portion, (B) a second outer portion, (C) a central portion longitudinally interposed between the outer portions, (D) a first set of laterally oriented openings formed through the first outer portion, and (E) a second set of laterally oriented openings formed through the second outer portion, and (iii) a plurality of discrete regions of bonding material securing the plurality of electrodes to the body, at least some of the discrete regions of the bonding material being disposed in the first set of laterally oriented openings, at least some of the discrete regions of the bonding material being disposed in the second set of laterally oriented openings.

### Example 30

A method comprising: positioning an electrode coaxially about an elongate body, the electrode having a pair of longitudinally opposed outer portions, each outer portion of the pair of longitudinally opposed outer portions defining an inner diameter, the elongate body defining an outer diameter, the inner diameter being larger than the outer diameter such that each outer portion of the pair of longitudinally opposed outer portions defines a respective gap with the elongate body, each outer portion of the pair of longitudinally opposed outer portions further defining a respective plurality of laterally oriented openings; flowing a bonding material into the plurality of laterally oriented openings and into the gaps; and allowing the bonding material to cure, thereby securing the electrode to the elongate body.

### V. Miscellaneous

Any of the instruments described herein may be cleaned and sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, hydrogen peroxide, peracetic acid, and vapor phase sterilization, either with or without a gas plasma, or steam.

It should be understood that any of the examples described herein may include various other features in addition to or in lieu of those described above. By way of example only, any of the examples described herein may also include one or more of the various features disclosed in any of the various references that are incorporated by reference herein.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Having shown and described various versions of the present invention, further adaptations of the methods and systems described herein may be accomplished by appropriate modifications by one skilled in the art without departing from the scope of the present invention. Several of such potential modifications have been mentioned, and others will be apparent to those skilled in the art. For instance, the examples, versions, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A ring electrode for pulsed field ablation comprising:
(a) a generally cylindrical member extending from one annular end portion to another annular end portion with a central portion including a first transition portion disposed between the central portion and the one annular portion and a second transition portion disposed between the central portion and the another annular end portion, the central portion including an inner diameter defining an inner surface about the longitudinal axis and an outer diameter defining the outer surface about the longitudinal axis, each of the annular end portions including an inner diameter smaller than the inner diameter of the central portion; and
(b) a plurality of circumferential cut outs disposed on one of the annular end portions or the transition portions.

2. The ring electrode of claim 1, in which the plurality of circumferential cut outs are disposed on the annular end portions, or on a portion of the annular end portion and the transition portion.

3. The ring electrode of claim 1, in which each of the cut out defines a circumferential slot having a width of approximately 0.20 mm and a length of approximately 0.40 mm.

4. An apparatus, comprising:
(a) an elongate flexible shaft having a proximal end and a distal end; and
(b) an end effector positioned at a distal end of the shaft, the end effector including:
(i) a body,
(ii) an electrode, the electrode defining one or more laterally oriented openings, the electrode including a pair of outer portions and a central portion longitudinally interposed between the outer portions, the bonding material being positioned over at least part of each outer portion of the pair of outer portions, and the at least one of the one or more laterally oriented openings in which the bonding material is disposed being positioned on an outer portion of the pair of outer portions, and
(iii) a bonding material securing the electrode to the body, a portion of the bonding material being disposed in at least one of the one or more laterally oriented openings.

5. The apparatus of claim 4, each outer portion of the pair of outer portions including a respective plurality of laterally oriented openings, the one or more laterally oriented openings of the electrode including the plurality of laterally oriented openings of the outer portions.

6. The apparatus of claim 4 or claim 5, each outer portion of the pair of outer portions defining a gap with an outer surface of the body, a portion of the bonding material being disposed in the gap.

7. The apparatus of claim 6, the gap being positioned adjacent to the at least one of the one or more laterally oriented openings in which the bonding material is disposed.

8. The apparatus of any of claims 4 to 7, each outer portion of the pair of outer portions having a first outer diameter, the central portion having a second outer diameter.

9. The apparatus of claim 8, the second outer diameter being larger than the first outer diameter.

10. The apparatus of claim 8, the electrode further including a pair of transition portions, each transition portion being longitudinally interposed between the central portion and a respective outer portion of the pair of outer portions, optionally the transition portions providing a smooth transition from the first outer diameter to the second outer diameter.

11. The apparatus of any of claims 8 to 10, the transition portions and the central portion being free of the bonding material, or the central portion being free of the bonding material.

12. The apparatus of any of claims 4 to 11, the one or more laterally oriented openings further including at least one irrigation opening configured to expel irrigation fluid from the electrode, optionally the body defining a lumen and a lateral port, the lumen and the lateral port being configured to communicate irrigation fluid to the electrode for expulsion via the at least one irrigation opening.

13. The apparatus of any of claims 4 to 12, the end effector further comprising a plurality of additional electrodes, the additional electrodes being longitudinally spaced apart from each other along the body, each electrode of the plurality of additional electrodes respectively defining one or more laterally oriented openings, the bonding material of the end effector further securing the additional electrodes to the body, respective portions of the bonding material being disposed in at least one of the one or more laterally oriented openings of the respective additional electrodes, optionally further comprising an actuator operable to drive the body between a first helical configuration and a second helical configuration.

14. The apparatus of any of claims 4 to 13, the bonding material comprising polyurethane, the body comprising poly(etherurethane), the electrode comprising a noble metal.

15. An apparatus, comprising:
(a) an elongate flexible shaft having a proximal end and a distal end; and
(b) an end effector positioned at a distal end of the shaft, the end effector including:
(i) a body,
(ii) a plurality of electrodes longitudinally spaced apart from each other along the body, each electrode of the plurality of electrodes including:
(A) a first outer portion,
(B) a second outer portion,
(C) a central portion longitudinally interposed between the outer portions,
(D) a first set of laterally oriented openings formed through the first outer portion, and
(E) a second set of laterally oriented openings formed through the second outer portion, and
(iii) a plurality of discrete regions of bonding material securing the plurality of electrodes to the body, at least some of the discrete regions of the bonding material being disposed in the first set of laterally oriented openings, at least some of the discrete regions of the bonding material being disposed in the second set of laterally oriented openings.
